# EUROPEAN PATENT SPECIFICATION

(11) **EP 3 668 480 B1**
(45) Date of publication and mention of the grant of the patent: **11.12.2024**
(21) Application number: 17826403.2
(22) Date of filing: 19.12.2017
(51) Int. Cl.: A61K 8/27, A61K 8/49, A61Q 11/00, A61Q 19/02, A61Q 19/08

(54) **PERSONAL CARE COMPOSITIONS INCLUDING NARINGIN:ZINC COMPLEXES AND METHODS FOR THE SAME**
KÖRPERPFLEGEZUSAMMENSETZUNGEN MIT NARINGIN: ZINKKOMPLEXEN UND VERFAHREN DAFÜR
COMPOSITIONS DE SOINS PERSONNELS COMPRENANT DES COMPLEXES DE NARINGINE : ZINC ET LEURS PROCÉDÉS D'UTILISATION

(30) Priority: 24.10.2017 WO PCT/US2017/057997
(43) Date of publication of application: 24.06.2020
(73) Proprietor: Colgate-Palmolive Company, New York, NY 10022 (US)
(72) Inventor: CHENG, Shujiang, Warren, New Jersey 07059 (US); NABI, Zeenat, Cranbury, New Jersey 08512 (US); DU-THUMM, Laurence D., Princeton NJ 08540 (US); WU, Qiang, Hillsborough, New Jersey 08844 (US); ONG, Jane, Franklin Park, New Jersey 08823 (US); SIOMYK, Halyna, Cliffside Park, New Jersey 07010 (US); TRIVEDI, Harsh, Hillsborough, New Jersey 08844 (US); MASTERS, James, Ringoes, New Jersey 08551 (US); CHENG, Chi-Yuan, Hillsborough, New Jersey 08844 (US); WU, Donghui, Bridgewater, New Jersey 08807 (US)
(74) Representative: Wichmann, Hendrik
(86) International application number: PCT/US2017/067407
(87) International publication number: WO 2019/083554

(56) References cited:
- EP-A1- 0 455 432
- EP-A1- 1 327 438
- WO-A1-2015/027308
- CN-A- 105 232 388
- SARRIA ANDRÉ LUCIO FRANCESCHINI ET AL: "Copper (II) and zinc (II) complexes with flavanone derivatives: Identification of potential cholinesterase inhibitors by on-flow assays", JOURNAL OF INORGANIC BIOCHEMISTRY, vol. 164, 17 September 2016 (2016-09-17), pages 141 - 149, XP029803062, ISSN: 0162-0134, DOI: 10.1016/J.JINORGBIO.2016.09.010
- OLFATA NIFE ET AL: "SYNTHESIS, CHARACTERIZATION, THEORETICAL STUDIES AND BIOLOGICAL ACTIVITIES OF NARINGIN METAL COMPLEXES REHAB ABDULMAHDI AL-HASSANI *", 1 January 2015 (2015-01-01), pages 20 - 9, XP055433944, Retrieved from the Internet <URL:http://www.tsijournals.com/articles/synthesis-characterization-theoretical-studies-and-biological-activities-of-naringin-metal-complexes.pdf> [retrieved on 20171211]

## Description

### CROSS-REFERENCE TO RELATED APPLICATIONS

This application claims priority to PCT Application No. PCT/US2017/057997, which was filed October 24, 2017.

### BACKGROUND

Signs of aging often appear on the skin as, among others, fine lines and wrinkles, age spots, dryness, blotchy discolorations, and sagging. Similarly, signs of aging may also manifest in the hair as frizziness, dullness, and loss of hair. These signs of aging or damage to the skin and hair are often exacerbated by other factors, such as diet, stress, environmental pollution, sun exposure, contact with chemicals (e.g., household chemicals), and the like.

Numerous personal care products or compositions thereof have been developed for the skin and hair to prevent or treat the signs of aging. For example, conventional personal care products often incorporate one or more benefit or therapeutic agents, such as taurine, that may facilitate the regeneration or healing of the skin and hair to counter the damage or signs of aging. While many benefit agents have been identified and utilized, the search for new benefit agents exhibiting improved results is ongoing.

What is needed, then, are improved personal care products and compositions thereof, and methods for regenerating and preventing inflammation of skin with the same.
Cosmetic preparation for preventive and regenerative treatment of skinaging comprising ursolic acid, oleic acid and/or flavonoids, wherein said flavonoids, e.g., may consist of a mixture of rutin and naringin, and optionally further comprising various additional compounds including, e.g., organic zinc compounds, are described in EP 1 327 438 A1. The naringin derivative α-glycosyl naringin and its use in vitamin P-enriching agents, food, beverages, tobaccos, foods, pet foods pharmaceuticals for susceptive diseases, cosmetics and plastics are described in EP 0 455 432 A1.

### BRIEF SUMMARY

This summary is intended merely to introduce a simplified summary of some aspects of one or more implementations of the present disclosure. Further areas of applicability of the present disclosure will become apparent from the detailed description provided hereinafter. This summary is not an extensive overview, nor is it intended to identify key or critical elements of the present teachings, nor to delineate the scope of the disclosure. Rather, its purpose is merely to present one or more concepts in simplified form as a prelude to the detailed description below.

The present invention provides a medical use of a personal care composition including a carrier and a naringin:zinc complex represented by chemical formula (I) as defined in the claims. The naringin:zinc complex has a 2:1 naringin to zinc molar ratio.

The naringin:zinc complex may have a melting point greater than or equal to 230°C, greater than or equal to 250°C, or greater than or equal to 280°C.

In at least one embodiment, the naringin:zinc complex may have a diffusion coefficient of from about 2.8E-11 m²/s to about 3.2E-11 m²/s in dimethyl sulfoxide and at 25°C.

In at least one embodiment, the naringin:zinc complex may be present in an amount of from about 0.01 weight % to about 5 weight %, preferably about 0.5 weight % to about 4.5 weight %, more preferably about 1.6 weight % to about 3.4 weight %, based on a total weight of the personal care composition.

In at least one embodiment, the naringin:zinc complex may be dispersed in the carrier.

In at least one embodiment, the personal care composition may be an antiperspirant, a deodorant, a body wash, a shower gel, a soap, a face wash, a shampoo, a hair conditioner, a lotion, a moisturizer, a serum, a spot treatment, or a cosmetic.

In at least one embodiment, the personal care composition may be a skin care product, and the carrier may include at least one of surfactants, conditioning agents, moisturizers, sunscreens, UV absorbers, antioxidants, enzymes and other proteins, vitamins, antibacterial agents, odor reducing agents, steroids, anti-inflammatory agents, naturally and non-naturally occurring humectants, skin lipid fluidizers, occlusive agents, amino acids, physical and chemical exfoliants, skin whiteners, anti-aging, antiperspirant actives, and mixtures thereof.

In at least one embodiment, the personal care composition may be a hair care product, and the carrier may include at least one of surfactants, colorants, denaturants, film forming polymers, conditioning agents, fixatives, hair color stabilizers, anti-inflammatory agents, antioxidants, moisturizers, enzymes and other proteins, vitamins, antidandruff agents, sunscreen agents, and mixtures thereof.

In at least one embodiment, the personal care composition may be an antiperspirant or a deodorant, and the carrier may include at least one of fragrances, alcohols, antimicrobial agents, antiperspirant salts, odor reducing agents, moisturizers, and mixtures thereof.

In at least one embodiment, the personal care composition may be a cleansing composition, and the carrier may include at least one of fragrances, essential oils, emulsifying agents, thickening agents, colorants, surfactants, natural actives, therapeutic actives, stain prevention actives, antimicrobial agents, vitamins, natural extracts, amino acids, enzymes and other proteins, abrasives, odor control agents, conditioning agents, moisturizers, humectants, occlusive agents, skin lipid fluidizers, lipophilic actives, hydrophilic materials, pearlizers, opacifying agents, and mixtures thereof.

In at least one embodiment, the carrier is anhydrous.

In at least one embodiment, the carrier is hydrophilic.

In at least one embodiment, the carrier is hydrophobic.

Embodiments of the disclosure also provide the personal care composition for use in a method for preventing or treating inflammation in or on skin of a subject as defined in the claims. The method may include contacting any of the personal care compositions disclosed herein with surfaces of the skin of the subject in need thereof.

Embodiments of the disclosure also provide the personal care composition for use in a method for promoting or facilitating cell repair in or on skin of a subject as defined in the claims. The method may include contacting any of the personal care compositions disclosed herein with surfaces of the skin of the subject in need thereof.

The personal care composition can also be used for whitening skin of a subject. Such use may include contacting any of the personal care compositions disclosed herein with surfaces of the skin of the subject in need thereof.

A method for preparing a personal care composition may include contacting a naringin:zinc complex with a carrier.

In at least one embodiment, the personal care composition is an antiperspirant, a deodorant, a body wash, a shower gel, a soap, a face wash, a shampoo, a hair conditioner, a lotion, or a cosmetic.

Further areas of applicability of the present disclosure will become apparent from the detailed description provided hereinafter. It should be understood that the detailed description and specific examples, while indicating some typical aspects of the disclosure, are intended for purposes of illustration only.

### DETAILED DESCRIPTION

The following description of various typical aspect(s) is merely exemplary in nature and is in no way intended to limit the disclosure, its application, or uses.

As used throughout, ranges are used as shorthand for describing each and every value that is within the range. Any value within the range may be selected as the terminus of the range.

Unless otherwise specified, all percentages and amounts expressed herein and elsewhere in the specification should be understood to refer to percentages by weight. The amounts given are based on the active weight of the material.

Additionally, all numerical values are "about" or "approximately" the indicated value, and take into account experimental error and variations that would be expected by a person having ordinary skill in the art. It should be appreciated that all numerical values and ranges disclosed herein are approximate values and ranges, whether "about" is used in conjunction therewith. It should also be appreciated that the term "about," as used herein, in conjunction with a numeral refers to a value that may be ± 0.01% (inclusive), ± 0.1% (inclusive), ± 0.5% (inclusive), ± 1% (inclusive) of that numeral, ± 2% (inclusive) of that numeral, ± 3% (inclusive) of that numeral, ± 5% (inclusive) of that numeral, ± 10% (inclusive) of that numeral, or ± 15% (inclusive) of that numeral. It should further be appreciated that when a numerical range is disclosed herein, any numerical value falling within the range is also specifically disclosed.

The present inventors have surprisingly and unexpectedly discovered that incorporating a naringin:zinc complex in personal care compositions may decease levels of an inflammation and irritation biomarker, namely IL-1a, in cells and tissues. The present inventors have also surprisingly and unexpectedly discovered that personal care compositions including a naringin:zinc complex may facilitate cell repair and regeneration.

### COMPOSITIONS

Compositions disclosed herein may be or include a personal care product or a personal care composition thereof. For example, compositions disclosed herein may be a personal care composition a personal care product, or form a portion of the personal care composition or the personal care product. Specifically, the compositions disclosed herein are personal care composition including a carrier and a naringin:zinc complex. The compositions including the carrier and an effective amount of the naringin:zinc complex are capable of or configured to provide one or more benefits to cells and/or tissue (e.g., skin). For example, the personal care compositions disclosed herein may be capable of or configured to facilitate, promote, or otherwise improve skin regeneration of a user or subject in need thereof. In another example, the personal care compositions disclosed herein may be capable of or configured to facilitate, promote, or otherwise improve one or more anti-inflammatory responses of the skin of a user or subject in need thereof.

### Naringin:Zinc Complex

The personal care composition includes a naringin:zinc complex. The naringin:zinc complex has a naringin to zinc molar ratio of 2:1, and may be represented by chemical formula (I).

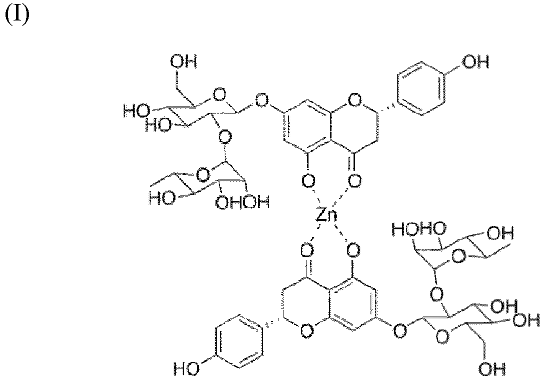

The naringin:zinc complex may have a melting point greater than or equal to 200°C. The naringin:zinc complex may also have a melting point less than or equal to about 305°C. For example, the melting point of the naringin:zinc complex may be from about 200°C, about 205°C, about 210°C, about 215°C, about 220°C, about 225°C, about 230°C, about 235°C, about 240°C, about 245°C, or about 250°C to about 255°C, about 260°C, about 265°C, about 270°C, about 275°C, about 280°C, about 285°C, about 290°C, about 295°C, about 300°C, or about 305°C. In another example, the melting point of the naringin:zinc complex may be from about 200°C to about 305°C, about 205°C to about 300°C, about 210°C to about 295°C, about 215°C to about 290°C, about 220°C to about 285°C, about 225°C to about 280°C, about 230°C to about 275°C, about 235°C to about 270°C, about 240°C to about 265°C, about 245°C to about 260°C, or about 250°C to about 255°C. In yet another example, the naringin:zinc complex may have a melting point greater than or equal to 200°C, greater than or equal to 205°C, greater than or equal to 210°C, greater than or equal to 215°C, greater than or equal to 220°C, greater than or equal to 225°C, greater than or equal to 230°C, greater than or equal to 235°C, greater than or equal to 240°C, greater than or equal to 245°C, or greater than or equal to 250°C to greater than or equal to 255°C, greater than or equal to 260°C, greater than or equal to 265°C, greater than or equal to 270°C, greater than or equal to 275°C, greater than or equal to 280°C, greater than or equal to 285°C, greater than or equal to 290°C, greater than or equal to 295°C, greater than or equal to 300°C, or greater than or equal to 305°C. In an exemplary implementation, the naringin:zinc complex has a melting point greater than or equal to 230°C.

The naringin:zinc complex may have a diffusion coefficient in dimethyl sulfoxide (DMSO) at 25°C of from about 2.3E-11 m²/s to about 3.2E-11 m²/s. For example, the naringin:zinc complex may have a diffusion coefficient in dimethyl sulfoxide (DMSO) at 25°C of from about 2.3E-11 m²/s, about 2.4E-11 m²/s, about 2.5E-11 m²/s, about 2.6E-11 m²/s, or about 2.7E-11 m²/s to about 2.8E-11 m²/s, about 2.9E-11 m²/s, about 3.0E-11 m²/s, about 3.1E-11 m²/s, or about 3.2E-11 m²/s. In another example, the naringin:zinc complex may have a diffusion coefficient in dimethyl sulfoxide (DMSO) at 25°C of from about 2.3E-11 m²/s to about 3.2E-11 m²/s, about 2.4E-11 m²/s to about 3.1E-11 m²/s, about 2.5E-11 m²/s to about 3.0E-11 m²/s, about 2.6E-11 m²/s to about 2.9E-11 m²/s, or about 2.7E-11 m²/s to about 2.8E-11 m²/s.

The naringin:zinc complex is present in the personal care composition in an effective amount or a therapeutically effective amount. As used herein, the expression or term "effective amount" may refer to an amount of the naringin:zinc complex sufficient to elicit a response (e.g., biological, medical, etc.) of a tissue, system, animal, or human that is being sought. For example, the naringin:zinc complex may be present in the personal care composition in an effective amount to initiate, improve, or otherwise facilitate skin regeneration. In another example, the naringin:zinc complex may be present in the personal care composition in an effective amount to provide an anti-inflammatory effect to a tissue (e.g., skin), system, animal, or human. In yet another example, the naringin:zinc complex may be present in the personal care composition in an effective amount to provide antioxidant, whitening, and/or collagen-boosting effects to a tissue (e.g., skin), system, animal, or human.

In at least one implementation, the naringin:zinc complex may be present in the personal care composition in an amount of from about 0.01 weight % to about 5 weight %, based on a total weight of the personal care composition. For example, the naringin:zinc complex may be present in the personal care composition in an amount of from about 0.01 weight %, about 0.1 weight %, about 0.2 weight %, about 0.3 weight %, about 0.4 weight %, about 0.5 weight %, about 0.6 weight %, about 0.7 weight %, about 0.8 weight %, about 0.9 weight %, about 1 weight %, about 1.2 weight %, about 1.4 weight %, about 1.6 weight %, about 1.8 weight %, about 2 weight %, about 2.2 weight %, or about 2.4 weight % to about 2.6 weight %, about 2.8 weight %, about 3 weight %, about 3.2 weight %, about 3.4 weight %, about 3.6 weight %, about 3.8 weight %, about 4 weight %, about 4.1 weight %, about 4.2 weight %, about 4.3 weight %, about 4.4 weight %, about 4.5 weight %, about 4.6 weight %, about 4.7 weight %, about 4.8 weight %, about 4.9 weight %, or about 5 weight %, based on a total weight of the personal care composition. In another example, the naringin:zinc complex may be present in the personal care composition in an amount of from about 0.01 weight % to about 5 weight %, about 0.1 weight % to about 4.9 weight %, about 0.2 weight % to about 4.8 weight %, about 0.3 weight % to about 4.7 weight %, about 0.4 weight % to about 4.6 weight %, about 0.5 weight % to about 4.5 weight %, about 0.6 weight % to about 4.4 weight %, about 0.7 weight % to about 4.3 weight %, about 0.8 weight % to about 4.2 weight %, about 0.9 weight % to about 4.1 weight %, about 1 weight % to about 4 weight %, about 1.2 weight % to about 3.8 weight %, about 1.4 weight % to about 3.6 weight %, about 1.6 weight % to about 3.4 weight %, about 1.8 weight % to about 3.2 weight %, about 2 weight % to about 3 weight %, about 2.2 weight % to about 2.8 weight %, or about 2.4 weight % to about 2.6 weight %, based on a total weight of the personal care composition

The naringin:zinc complex may be prepared by combining, mixing, reacting, or otherwise contacting naringin and zinc (i.e., zinc ions) or a source of zinc with one another in a suitable medium or solvent. For example, the naringin:zinc complex may be prepared by contacting the naringin and a source of zinc with one another in an organic solvent. Illustrative organic solvents may be or include, but are not limited to, methanol, ethanol, propanol, methylene glycol, ethylene glycol, propylene glycol, and the like, and mixtures or combinations thereof. In another example, the naringin:zinc complex may be prepared by contacting the naringin and the source of zinc with one another in an aqueous solvent, such as water.

The naringin:zinc complex may be prepared by heating the mixture of the naringin, the source of zinc, and/or the suitable medium or solvent to a temperature of from about 20°C to about 80°C. For example, the naringin:zinc complex may be prepared by heating the mixture of the naringin, the source of zinc, and the suitable medium or solvent to a temperature of from about 20°C, about 25°C, about 30°C, about 35°C, about 40°C, or about 45°C to about 55°C, about 60°C, about 65°C, about 70°C, about 75°C, or about 80°C. In another example, the naringin:zinc complex may be prepared by heating the mixture of the naringin, the source of zinc, and the suitable medium or solvent to a temperature of from about 20°C to about 80°C, about 25°C to about 75°C, about 30°C to about 70°C, about 35°C to about 65°C, about 40°C to about 60°C, or about 45°C to about 55°C. In yet another example, the naringin:zinc complex may be prepared by heating the mixture of the naringin, the source of zinc, and the suitable medium or solvent to a temperature of at least 20°C, at least 25°C, at least 30°C, at least 35°C, at least 40°C, at least 45°C, at least 50°C, at least 55°C, at least 60°C, at least 65°C, at least 70°C, or at least 75°C.

The pH of the mixture including the naringin, the source of zinc, and/or the suitable medium or solvent may be adjusted. For example, the pH of the mixture including the naringin, the source of zinc, and/or the suitable medium or solvent may be increased or decreased through the addition of a base or an acid, respectively. In at least one implementation, the pH of the mixture including the naringin, the source of zinc, and/or the suitable medium or solvent may be adjusted to about 7, about 8, about 9, about 10, or about 11. In another example, the naringin-zinc complex may be prepared in a pH of about 7, about 8, about 9, about 10, or about 11.

The zinc or zinc ions may be provided by one or more zinc ion sources. For example, the zinc ions may be provided by one or more zinc salts. Illustrative zinc ion sources may be or include, but are not limited to, zinc oxide, zinc acetate, zinc chloride, zinc lactate, tetrabasic zinc chloride, zinc carbonate, zinc nitrate, zinc citrate, zinc bisglycinate, zinc phosphate, and the like, and mixtures or combinations thereof. It should be appreciated that zinc oxide and tetrabasic zinc chloride are insoluble in water, but readily form complexes with naringin in water upon the addition of heat. In a preferred implementation, the zinc ion source includes at least one of zinc acetate, zinc oxide, zinc chloride, zinc lactate, zinc citrate, zinc nitrate, and mixtures or combinations thereof.

### Carrier or Excipient

The personal care composition may include the naringin:zinc complex dispersed in, mixed with, dissolved in, combined with, or otherwise contacted with the carrier. In at least one implementation, the carrier may be capable of or configured to store, entrain, or otherwise contain the naringin:zinc complex, and deliver the naringin:zinc complex. It should be appreciated that the components or contents of the carrier and the respective amount of each of the components of the carrier may be at least partially determined by the type or use of the personal care product or the composition thereof. Illustrative personal care products or compositions thereof that may include the naringin:zinc complex include, but are not limited to, antiperspirants, deodorants, body washes, shower gels, soaps, including bar soaps and liquid soaps (e.g., liquid hand soaps), face washes, shampoos, hair conditioners, lotions, moisturizers, serums, spot treatments, cosmetics, and the like.

In at least one implementation, the personal care product or the composition thereof may be a skin care product. Illustrative skin care product may be or include, but are not limited to, a lotion, a cosmetic, a sunscreen, and the like. The carrier of the skin care product may include, but is not limited to, any one or more of surfactants, conditioning agents, moisturizers, sunscreens, UV absorbers, antioxidants, enzymes and other proteins, vitamins, antibacterial agents, odor reducing agents, steroids, anti-inflammatory agents, naturally and non-naturally occurring humectants, skin lipid fluidizers, occlusive agents, amino acids, physical and chemical exfoliants, skin whiteners, anti-aging, antiperspirant actives, and the like, and mixtures thereof.

In at least one implementation, the personal care product or the composition thereof may be a hair care product. Illustrative hair care products may be or include, but are not limited to, a shampoo (e.g., dry and wet shampoo), a conditioner (e.g., leave in conditioner), hair spray, gels, and the like. The carrier for the hair care product may include, but is not limited to, any one or more of surfactants, colorants, denaturants, film forming polymers, conditioning agents, fixatives, hair color stabilizers, anti-inflammatory agents, antioxidants, moisturizers, enzymes and other proteins, vitamins, antidandruff agents, sunscreen agents, and mixtures thereof. Illustrative conditioning agents may include, but are not limited to, any one or more of silicones (e.g., silicone oils, cationic silicones, silicone gums, high refractive silicones, silicone resins, etc.), and organic conditioning oils (e.g., hydrocarbon oils, polyolefins, fatty esters, etc.). Illustrative anti-dandruff agents may include, but are not limited to, pyridinethione salts, selenium sulfide, particulate sulfur, and the like, and mixtures thereof. Illustrative antiinflammatories may include, but are not limited to, glucocorticoids, nonsteroidal antiinflammatories, and the like, and mixtures or combinations thereof. Illustrative colorants may include, but are not limited to, non-oxidative dyes, such as "direct action dyes," metallic dyes, metal chelate dyes, fiber/fibre reactive dyes, other synthetic and natural dyes, and the like, and mixtures or combinations thereof. Illustrative sunscreen agents may include, but are not limited to, 2-ethylhexyl p-methoxycinnamate, 2-ethylhexyl N,N-dimethyl-p-aminobenzoate, p-aminobenzoic acid, 2-phenylbenzimidazole-5-sulfonic acid, octocrylene, oxybenzone, homomenthyl salicylate, octyl salicylate, 4,4'-methoxy-t-butyldibenzoylme-thane, 4-isopropyl dibenzoylmethane, 3-benzylidene camphor, 3-(4-methylbenzylidene) camphor, titanium dioxide, zinc oxide, silica, iron oxide, and the like, and mixtures or combinations thereof. Illustrative vitamins may include, but are not limited to, Vitamin E, panthenol, and the like, and mixtures or combinations thereof.

In at least one implementation, the personal care product or the composition thereof may be an antiperspirant or deodorant. The carrier for the antiperspirant or deodorant may include, but is not limited to, any one or more of fragrances, alcohols, antimicrobial agents, antiperspirant salts, odor reducing agents, moisturizers, other components as discussed above for skin and hair care compositions, and mixtures or combinations thereof. Illustrative antimicrobial agents may include, but are not limited to, primary oleamine salt of piroctone, metal salts of piroctone acid (such as aluminum, sodium, potassium, zirconium, calcium and zinc metal salts), triclosan, zinc phenol sulfonate, heavy metal salts of 1-hydroxy pyridinethione (such as zinc pyrithione, magnesium pyrithione, and aluminum pyrithione), bacteriostatic quaternary ammonium compounds (such as cetyl-trimethyl ammonium bromide, cetyl pyridinium chloride, benzethonium chloride, and sodium N-lauryl-sarcosine), carbonate and bicarbonate salts, and the like, and mixtures or combinations thereof. Illustrative antiperspirant salts may include, but are not limited to, poly-valent metal salts and complexes thereof, such as aluminum halides, aluminum hydroxy-halides, zirconyl oxyhalides, zirconyl hydroxy-halides, zinc compounds, such as zinc phenyl sulfonate, zinc glycinate and zinc pyrithione, and the like, and mixtures or combinations thereof. Illustrative complexes may be or include, but are not limited to, amino acids (e.g., glycine) forming antiperspirant actives commonly known as "ZAG," including aluminum, zirconium, and chlorine having an Al:Zr ratio of about 1.67 to about 12.5 and a Metal:Cl ratio of about 0.73 to about 1.93. Illustrative odor reducing agents may include, but are not limited to, sulfur precipitating agents, such as copper gluconate, zinc citrate, zinc gluconate, and the like, and mixtures or combinations thereof.

In at least one implementation, the personal care product or the composition thereof may be a personal hand and/or body cleansing composition or a personal hand and/or body conditioning composition. Illustrative personal hand and/or body cleansing or conditioning compositions may include, but are not limited to, liquid soaps, bar soaps, body washes, lotions, and the like. The carrier for the personal hand and/or body cleansing composition or the personal hand and/or body conditioning composition may include, but is not limited to, any one or more of fragrances, essential oils, emulsifying agents, thickening agents, colorants, surfactants, natural actives, therapeutic actives, stain prevention actives, antimicrobial agents, vitamins, natural extracts, amino acids, enzymes or other proteins, abrasives, odor control agents, conditioning agents, moisturizers, humectants, occlusive agents, skin lipid fluidizers, lipophilic actives, hydrophilic materials, pearlizers, opacifying agents, and the like, and mixtures or combinations thereof, in addition to the other carrier components as discussed above.

The carrier may be hydrophilic or hydrophobic. The carrier may be anhydrous. The carrier may be a liquid or a solid at room temperature. The carrier may have a viscosity of from about 2,000 centipoise (cP) to about 100,000 cP. For example, the carrier for a shower gel may have a viscosity of from about 2,000 cP to about 16,000 cP. In another example, the carrier for a lotion may have a viscosity of from about 10,000 cP to about 100,000 cP. Accordingly, it should be appreciated that the viscosity of the carrier may vary and may at least partially depend on the type of personal care composition.

Unless otherwise specifically identified, the ingredients for use in the compositions and formulations are preferably cosmetically acceptable ingredients. By "cosmetically acceptable" is meant suitable for use in a formulation for topical application to human skin. A cosmetically acceptable excipient, for example, is an excipient which is suitable for external application in the amounts and concentrations contemplated in the formulations used according to this invention, and includes for example, excipients which are "Generally Recognized as Safe" (GRAS) by the United States Food and Drug Administration.

### METHODS

Methods for preparing a naringin:zinc complex having a 2:1 mole ratio of naringin to zinc are described herein which may include contacting naringin and zinc or a source of zinc in a suitable medium, such as water or an organic solvent. The method may also include heating the naringin and zinc or the source of zinc in the suitable medium. The method may further include adjusting the pH of the suitable medium including the naringin and zinc or the source of zinc.

Also, methods for preparing a personal care composition are described herein which may include contacting a naringin:zinc complex with a carrier.

Also, methods for reducing or treating inflammation of tissue (e.g., skin) are described herein which may include contacting the tissue (e.g., surface of the skin) with a naringin:zinc complex. The method may also include combining or otherwise contacting a naringin:zinc complex with a carrier to prepare a personal care composition, and contacting the tissue with the personal care composition.

Also, methods for reducing irritation of tissue (e.g., skin) are described herein which may include contacting the tissue (e.g., surface of the skin) with a naringin:zinc complex. The method may also include combining or otherwise contacting a naringin:zinc complex with a carrier to prepare a personal care composition, and contacting the tissue with the personal care composition.

Also, methods for promoting or facilitating cell repair or regeneration of tissue (e.g., skin) are described herein which may include contacting the tissue (e.g., surface of the skin) with a naringin:zinc complex. The method may also include combining or otherwise contacting a naringin:zinc complex with a carrier to prepare a personal care composition, and contacting the tissue with the personal care composition.

The present disclosure further provides a personal care composition including a carrier and a naringin:zinc complex for use in reducing or treating inflammation of skin as defined in the claims.

The personal care composition including a carrier and a naringin:zinc complex may be used for reducing irritation of tissue (e.g., skin).

The present disclosure further provides a personal care composition including a carrier and a naringin:zinc complex for use in promoting or facilitating repair or regeneration of skin as defined in the claims.

Further, a method of making a personal care composition for reducing or treating inflammation of tissue is described herein which may include combining or otherwise contacting a naringin:zinc complex with a carrier to prepare the personal care composition.

Further, a method of making a personal care composition for reducing irritation of tissue is described herein which may include combining or otherwise contacting a naringin:zinc complex with a carrier to prepare the personal care composition.

Further, a method of making a personal care composition for promoting or facilitating cell repair or regeneration of tissue is described herein which may include combining or otherwise contacting a naringin:zinc complex with a carrier to prepare the personal care composition.

### EXAMPLES

The examples and other implementations described herein are exemplary and not intended to be limiting.

### Example 1

The anti-inflammatory properties of the naringin:zinc complex was evaluated by conducting an IL-1a assay using HaCaT cells. To evaluate the anti-inflammatory properties, HaCaT cells were grown into 80% confluency and pretreated for 24 hours with 1 mL of each of the following samples: (1) a culture media (control); (2) 0.001% naringin:zinc complex; and (3) 0.001% naringin alone. To prepare the 0.001% naringin-zinc complex and the 0.001% naringin alone, each of the naringin:zinc complex and the naringin were prepared in stock solutions of 1% DMSO/PBS and diluted to the target concentration using culture media.

After pretreating the HaCaT cells, the cells were stressed with 0.01% SDS for 15 minutes before adding another 1 mL of each of the samples (1)-(3). Each of the HaCaT cells were used to prepare cell supernatants, which were used for IL-1a assays with IL-1a ELISA kits. The results of the IL-1a assay are summarized in Table 1.

**Table 1**

| **IL-1a Assay**¹ | | |
|---|---|---|
| **Sample** | **pg/mL** | **Std Dev** |
| Control (1) Prior to SDS Stress | 1.7 | 0.6 |
| Control (1) | 8.3 | 1.0 |
| 0.001% Naringin:Zinc Complex (2) | 5.2 | 1.9 |
| Naringin (3) | 6.6 | 1.3 |

| | | |
|---|---|---|
| ¹ IL-1a levels were normalized by cell viability | | |

It should be appreciated that IL-1a is one of the main biomarkers for inflammation/irritation. It should further be appreciated that SDS is a well-known inducer for skin irritation. As indicated in Table 2, treatment with 0.01% SDS on the control resulted in a four-fold increase in IL-1a levels from 1.7 pg/mL to 8.3 pg/mL. As further illustrated in Table 2, the addition of the naringin:zinc complex prior to and after SDS stress treatment resulted in a reduction of the inflammation biomarker as compared to the control. The results also surprisingly and unexpectedly indicate that the naringin:zinc complex exhibited relatively lower IL-1a as compared to the naringin alone (3).

### Example 2

The soothing properties or benefits of the naringin:zinc complex were evaluated in an exemplary carrier. Particularly, the soothing properties were evaluated by incorporating the naringin:zinc complex in an amount of 0.5% in a skin cleansing carrier. EpiDerm^{™} tissue obtained from MatTek Corporation was treated for 1 hour with either (4) the skin cleansing carrier alone; or (5) the skin cleansing carrier including the naringin:zinc complex. After treatment, each of the tissue samples were washed six to seven times with phosphate buffered saline (PBS) and incubated in a cultured media for at least 18 hours. The supernatants were sampled for IL-1a levels, and the results are summarized in Table 2.

**Table 2**

| **IL-1a Assay of EpiDerm^{™} tissue ²** | | |
|---|---|---|
| **Sample** | **pg/mL** | **Std Dev** |
| Control | 32.8 | 1.0 |
| Carrier (4) | 76.8 | 5.9 |
| Carrier + Naringin:Zinc Complex (5) | 53.1 | 12.6 |

| | | |
|---|---|---|
| ² IL-1a levels were normalized by Total Protein Concentration | | |

As illustrated in Table 2, the naringin:zinc complex exhibited significantly lower IL-1a levels, which represented irritation potential. The significant reduction of the IL-1a, which were both surprising and unexpected, further confirmed the anti-inflammatory properties of the complex.

### Example 3

The cell regeneration function or properties of the naringin:zinc complex were evaluated via a scratch assay using human epidermal keratinocyte cells obtained from Thermal Fisher Scientific of Gaithersburg, MD. Particularly, monolayers of keratinocyte cells were gown to full confluency in separate wells, and each well was scratched with a 1000 µl tip to create a cell-free gap. Each of the wells with the cell-free gap was then washed with 1 mL of PBS twice to remove any dead floating cells. Each of the wells were treated/incubated with 1 mL of each of the following samples: (6) culture media (control); (7) 0.001% naringin:zinc complex; (8) 0.001% naringin alone; (9) 0.000065% zinc oxide (ZnO); and (10) naringin and zinc oxide mixture. Distance measurements of the gap were taken every 24 hours until the gaps fully closed. The distance measurements of each of the respective gaps before and after treatment/incubation were taken using a microscope (OLYMPUS^{®} IX71), and the results are summarized in Table 3.

**Table 3**

| **Cell Migration of Keratinocytes (% Active)** | | |
|---|---|---|
| **Sample** | **pg/mL** | **P value vs. Control** |
| Control (6) | 47 | -- |
| 0.001% Naringin:Zinc Complex (7) | 81 | 0.0001 |
| 0.001% Naringin (8) | 58 | 0.7457 |
| 0.000065% ZnO (9) | 59 | 0.0454 |
| Naringin + ZnO mixture (10) | 63 | 0.0405 |

Table 3 illustrates the percentage of gap closure versus the starting gap distance. It should be appreciated that a relatively higher value of the percentage of gap closure indicates a relatively greater repair or regeneration function. While each of the samples, except naringin alone (8), demonstrated statistically significant repair or regeneration as compared to the control (6) (p < 0.05), it was surprisingly and unexpectedly discovered that the naringin:zinc complex exhibited a relatively greater and statistically significant increase in the repair or regeneration function as compared to the remaining samples. It was further surprisingly and unexpectedly discovered that the naringin and zinc oxide mixture (10), including the naringin and the zinc oxide in the same molar ratio as the naringin-zinc complex (7), exhibited relatively lower repair activity as compared to the naringin-zinc complex (7). These results supported the synergistic relationship between the naringin and zinc in the naringin:zinc complex (7) and are nothing short of surprising and unexpected.

## Claims

1. A personal care composition for use in preventing or treating inflammation in skin, promoting or facilitating cell repair in skin, of a subject in need thereof,
wherein the personal care composition comprises:
a cosmetically acceptable carrier; and
a naringin:zinc complex having a 2:1 naringin to zinc molar ratio represented by chemical formula (I):.

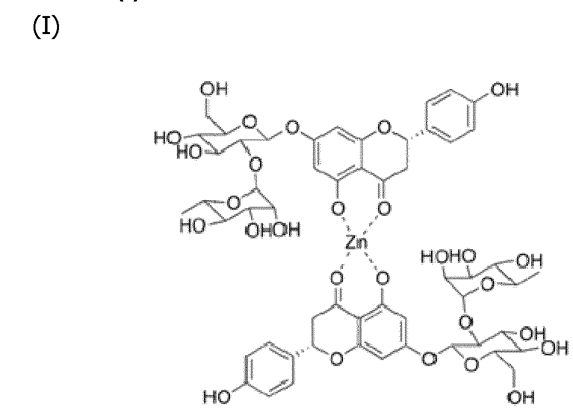

2. The personal care composition for use according to claim 1, wherein the naringin:zinc complex has a diffusion coefficient of from 2.8E-11 m2/s to 3.2E-11 m2/s in dimethyl sulfoxide and at 25°C.

3. The personal care composition for use according to one of the preceding claims, wherein the naringin:zinc complex is present in an amount of from 0.01 weight % to 5 weight %, preferably from 0.5 weight % to 4.5 weight %, more preferably from 1.6 weight % to 3.4 weight %, based on a total weight of the personal care composition.

4. The personal care composition for use according to one of the preceding claims, wherein the naringin:zinc complex is dispersed in the carrier.

5. The personal care composition for use according to one of the preceding claims, wherein the personal care composition is a skin care product, and wherein the carrier comprises at least one of surfactants, conditioning agents, moisturizers, sunscreens, UV absorbers, antioxidants, enzymes and other proteins, vitamins, antibacterial agents, odor reducing agents, steroids, anti-inflammatory agents, naturally and non-naturally occurring humectants, skin lipid fluidizers, occlusive agents, amino acids, physical and chemical exfoliants, skin whiteners, anti-aging, antiperspirant actives, and mixtures thereof.

6. The personal care composition for use according to one of the preceding claims, wherein the personal care composition is a hair care product, and wherein the carrier comprises at least one of surfactants, colorants, denaturants, film forming polymers, conditioning agents, fixatives, hair color stabilizers, anti-inflammatory agents, antioxidants, moisturizers, enzymes and other proteins, vitamins, antidandruff agents, sunscreen agents, and mixtures thereof.

7. The personal care composition for use according to one of the preceding claims, wherein the personal care composition is an antiperspirant or a deodorant, and wherein the carrier comprises at least one of fragrances, alcohols, antimicrobial agents, antiperspirant salts, odor reducing agents, moisturizers, and mixtures thereof.

8. The personal care composition for use according to one of the preceding claims, wherein the personal care composition is a cleansing composition, and wherein the carrier comprises at least one of fragrances, essential oils, emulsifying agents, thickening agents, colorants, surfactants, natural actives, therapeutic actives, stain prevention actives, antimicrobial agents, vitamins, natural extracts, amino acids, enzymes and other proteins, abrasives, odor control agents, conditioning agents, moisturizers, humectants, occlusive agents, skin lipid fluidizers, lipophilic actives, hydrophilic materials, pearlizers, opacifying agents, and mixtures thereof.

9. The personal care composition for use according to one of the preceding claims, wherein the carrier is anhydrous.

10. The personal care composition for use according to one of the preceding claims, wherein the carrier is hydrophilic or hydrophobic.

11. The personal care composition for use according to one of the preceding claims, wherein the carrier has a viscosity of from 2,000 cP to 100,000 cP, 2,000 cP to 16,000 cP, or 10,000 cP to 100,000 cP.

12. The personal care composition for use according to one of the claims 1 to 11, for preventing or treating inflammation in skin of a subject, comprising contacting the personal care composition with surfaces of the skin of the subject in need thereof.

13. The personal care composition for use according to one of the claims 1 to 11, for promoting or facilitating cell repair in skin of a subject, comprising contacting the personal care composition with surfaces of the skin of the subject in need thereof.

14. The personal care composition for use according to one of the preceding claims, wherein the personal care composition is an antiperspirant, a deodorant, a body wash, a shower gel, a soap, a face wash, a shampoo, a hair conditioner, a lotion, a moisturizer, a serum, a spot treatment or a cosmetic.

## Patentansprüche

1. Körperpflegezusammensetzung zur Verwendung bei der Vorbeugung oder Behandlung von Entzündungen in der Haut, zur Förderung oder Erleichterung der Zellreparatur in der Haut eines Individuums, das diese benötigt,
wobei die Körperpflegezusammensetzung folgendes umfasst:
einen kosmetisch akzeptablen Träger; und
einen Naringin:Zink-Komplex mit einem molaren Verhältnis von 2:1 von Naringin zu Zink, dargestellt durch die chemische Formel (I):.

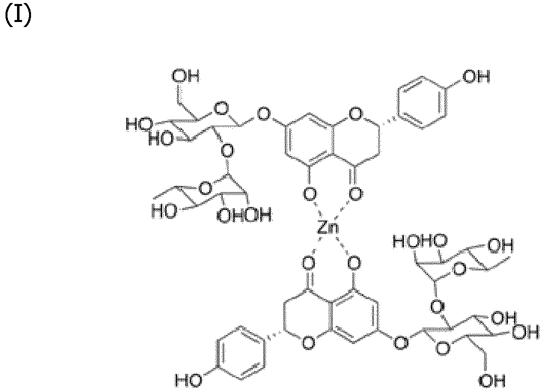

2. Körperpflegezusammensetzung zur Verwendung nach Anspruch 1, wobei der Naringin:Zink-Komplex einen Diffusionskoeffizienten von 2,8E-11 m²/s bis 3,2E-11 m²/s in Dimethylsulfoxid und bei 25°C aufweist.

3. Körperpflegezusammensetzung zur Verwendung nach einem der vorhergehenden Ansprüche, wobei der Naringin:Zink-Komplex in einer Menge von 0,01 Gew.-% bis 5 Gew.-%, vorzugsweise von 0,5 Gew.-% bis 4,5 Gew.-%, besonders bevorzugt von 1,6 Gew.-% bis 3,4 Gew.-%, bezogen auf das Gesamtgewicht der Körperpflegezusammensetzung, vorliegt.

4. Körperpflegezusammensetzung zur Verwendung nach einem der vorhergehenden Ansprüche, wobei der Naringin:Zink-Komplex in dem Träger dispergiert ist.

5. Körperpflegezusammensetzung zur Verwendung nach einem der vorhergehenden Ansprüche, wobei die Körperpflegezusammensetzung ein Hautpflegeprodukt ist, und wobei der Träger mindestens eines der folgenden umfasst: Tenside, Konditionierungsmittel, Feuchtigkeitsspender, Sonnenschutzmittel, UV-Absorber, Antioxidantien, Enzyme und andere Proteine, Vitamine, antibakterielle Mittel, geruchsreduzierende Mittel, Steroide, entzündungshemmende Mittel, natürlich und nicht natürlich vorkommende Feuchthaltemittel, Hautlipiderflüssiger, Okklusionsmittel, Aminosäuren, physikalische und chemische Peelings, Hautaufheller, Anti-Aging- und Antitranspirant-Wirkstoffe und Mischungen davon.

6. Körperpflegezusammensetzung zur Verwendung nach einem der vorhergehenden Ansprüche, wobei die Körperpflegezusammensetzung ein Haarpflegeprodukt ist und wobei der Träger mindestens eines der folgenden umfasst: Tenside, Farbstoffe, Denaturierungsmittel, filmbildende Polymere, Konditionierungsmittel, Fixiermittel, Haarfarbstabilisatoren, entzündungshemmende Mittel, Antioxidantien, Feuchtigkeitsspender, Enzyme und andere Proteine, Vitamine, Antischuppenmittel, Sonnenschutzmittel und Mischungen davon.

7. Körperpflegezusammensetzung zur Verwendung nach einem der vorhergehenden Ansprüche, wobei die Körperpflegezusammensetzung ein Antitranspirant oder ein Deodorant ist und wobei der Träger mindestens eines der folgenden umfasst: Duftstoffe, Alkohole, antimikrobielle Mittel, Antitranspirantsalze, geruchsreduzierende Mittel, Feuchtigkeitsspender und Mischungen davon.

8. Körperpflegezusammensetzung zur Verwendung nach einem der vorhergehenden Ansprüche, wobei die Körperpflegezusammensetzung eine Reinigungszusammensetzung ist und wobei der Träger mindestens eines der folgenden umfasst: Duftstoffe, ätherische Öle, Emulgatoren, Verdickungsmittel, Farbstoffe, Tenside, natürliche Wirkstoffe, therapeutische Wirkstoffe, Wirkstoffe zur Verhinderung von Flecken, antimikrobielle Mittel, Vitamine, natürliche Extrakte, Aminosäuren, Enzyme und andere Proteine, Schleifmittel, Geruchskontrollmittel, Konditionierungsmittel, Feuchtigkeitsspender, Feuchthaltemittel, Okklusionsmittel, Hautlipidverflüssiger, lipophile Wirkstoffe, hydrophile Materialien, Perlglanzmittel, Trübungsmittel und Mischungen davon.

9. Körperpflegezusammensetzung zur Verwendung nach einem der vorhergehenden Ansprüche, wobei der Träger wasserfrei ist.

10. Körperpflegezusammensetzung zur Verwendung nach einem der vorhergehenden Ansprüche, wobei der Träger hydrophil oder hydrophob ist.

11. Körperpflegezusammensetzung zur Verwendung nach einem der vorhergehenden Ansprüche, wobei der Träger eine Viskosität von 2.000 cP bis 100.000 cP, 2.000 cP bis 16.000 cP oder 10.000 cP bis 100.000 cP aufweist.

12. Körperpflegezusammensetzung zur Verwendung nach einem der Ansprüche 1 bis 11 zur Vorbeugung oder Behandlung von Entzündungen in der Haut eines Individuums, umfassend das Inkontaktbringen der Körperpflegezusammensetzung mit Hautoberflächen des Individuums, das diese benötigt.

13. Körperpflegezusammensetzung zur Verwendung nach einem der Ansprüche 1 bis 11 zur Förderung oder Erleichterung der Zellreparatur in der Haut eines Individuums, umfassend das Inkontaktbringen der Körperpflegezusammensetzung mit Hautoberflächen des Individuums, das diese benötigt.

14. Körperpflegezusammensetzung zur Verwendung nach einem der vorhergehenden Ansprüche, wobei die Körperpflegezusammensetzung ein Antitranspirant, ein Deodorant, ein Körperwaschmittel, ein Duschgel, eine Seife, ein Gesichtswaschmittel, ein Shampoo, eine Haarspülung, eine Lotion, eine Feuchtigkeitsspender, ein Serum, eine Fleckenbehandlung oder ein Kosmetikum ist.

## Revendications

1. Composition de soins personnels pour une utilisation pour prévenir ou traiter l'inflammation de la peau, favoriser ou faciliter la réparation cellulaire de la peau, d'un sujet en ayant besoin,
dans laquelle la composition de soins personnels comprend :
un support cosmétiquement acceptable ; et
un complexe naringine:zinc ayant un rapport molaire naringine sur zinc de 2:1 représenté par la formule chimique (I) :

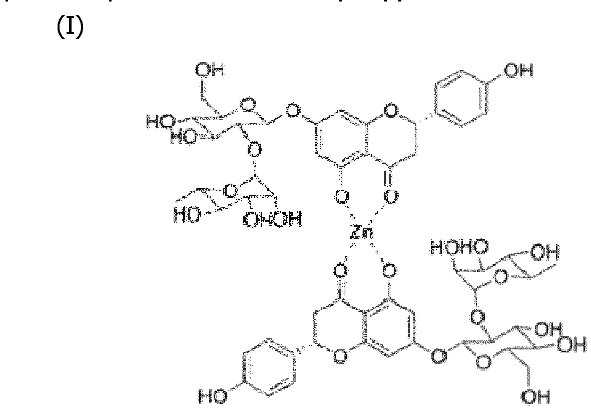

2. Composition de soins personnels pour une utilisation selon la revendication 1, dans laquelle le complexe naringine:zinc a un coefficient de diffusion de 2,8E-11 m2/s à 3,2E-11 m2/s dans le diméthylsulfoxyde et à 25 °C.

3. Composition de soins personnels pour une utilisation selon l'une des revendications précédentes, dans laquelle le complexe naringine:zinc est présent en une quantité de 0,01 % en poids à 5 % en poids, de préférence de 0,5 % en poids à 4,5 % en poids, plus préférablement de 1,6 % en poids à 3,4 % en poids, par rapport au poids total de la composition de soins personnels.

4. Composition de soins personnels pour une utilisation selon l'une des revendications précédentes, dans laquelle le complexe naringine:zinc est dispersé dans le support.

5. Composition de soins personnels pour une utilisation selon l'une des revendications précédentes, dans laquelle la composition de soins personnels est un produit de soins de la peau, et dans laquelle le support comprend au moins un élément parmi les tensioactifs, les agents de conditionnement, les hydratants, les écrans solaires, les absorbeurs d'UV, les antioxydants, les enzymes et autres protéines, les vitamines, les agents antibactériens, les agents de réduction des odeurs, les stéroïdes, les agents anti-inflammatoires, les humectants naturels et non naturels, les fluidifiants des lipides cutanés, les agents occlusifs, les acides aminés, les exfoliants physiques et chimiques, les agents de blanchiment pour la peau, les actifs anti-âge, anti-transpirants et les mélanges de ceux-ci.

6. Composition de soins personnels pour une utilisation selon l'une des revendications précédentes, dans laquelle la composition de soins personnels est un produit de soins capillaires, et dans laquelle le support comprend au moins un élément parmi les tensioactifs, les colorants, les dénaturants, les polymères filmogènes, les agents de conditionnement, les fixateurs, les stabilisateurs de couleur de cheveux, les agents anti-inflammatoires, les antioxydants, les hydratants, les enzymes et autres protéines, les vitamines, les agents antipelliculaires, les agents de protection solaire et les mélanges de ceux-ci.

7. Composition de soins personnels pour une utilisation selon l'une des revendications précédentes, dans laquelle la composition de soins personnels est un antitranspirant ou un déodorant, et dans laquelle le support comprend au moins un élément parmi les parfums, les alcools, les agents antimicrobiens, les sels anti-transpirants, les agents de réduction des odeurs, les hydratants et les mélanges de ceux-ci.

8. Composition de soins personnels pour une utilisation selon l'une des revendications précédentes, dans laquelle la composition de soins personnels est une composition nettoyante, et dans laquelle le support comprend au moins un élément parmi les parfums, les huiles essentielles, les agents émulsifiants, les agents épaississants, les colorants, les tensioactifs, les actifs naturels, les actifs thérapeutiques, les actifs de prévention des taches, les agents antimicrobiens, les vitamines, les extraits naturels, les acides aminés, les enzymes et autres protéines, les abrasifs, les agents de lutte contre les odeurs, les agents de conditionnement, les hydratants, les humectants, les agents occlusifs, les fluidifiants des lipides cutanés, les actifs lipophiles, les matières hydrophiles, les agents nacrants, les agents opacifiants et les mélanges de ceux-ci.

9. Composition de soins personnels pour une utilisation selon l'une des revendications précédentes, dans laquelle le support est anhydre.

10. Composition de soins personnels pour une utilisation selon l'une des revendications précédentes, dans laquelle le support est hydrophile ou hydrophobe.

11. Composition de soins personnels pour une utilisation selon l'une des revendications précédentes, dans laquelle le support a une viscosité de 2 000 cP à 100 000 cP, de 2 000 cP à 16 000 cP ou de 10 000 cP à 100 000 cP.

12. Composition de soins personnels pour une utilisation selon l'une des revendications 1 à 11, pour prévenir ou traiter une inflammation de la peau d'un sujet, comprenant la mise en contact de la composition de soins personnels avec des surfaces de la peau du sujet en ayant besoin.

13. Composition de soins personnels pour une utilisation selon l'une des revendications 1 à 11, pour favoriser ou faciliter la réparation cellulaire de la peau d'un sujet, comprenant la mise en contact de la composition de soins personnels avec des surfaces de la peau du sujet en ayant besoin.

14. Composition de soins personnels pour une utilisation selon l'une des revendications précédentes, dans laquelle la composition de soins personnels est un antitranspirant, un déodorant, un produit de lavage corporel, un gel douche, un savon, un produit de lavage du visage, un shampooing, un après-shampooing, une lotion, un hydratant, un sérum, un traitement localisé ou un produit cosmétique.
